# EUROPEAN PATENT APPLICATION

(11) **EP 4 424 225 A1**
(43) Date of publication of application: **04.09.2024**
(21) Application number: 22856988.5
(22) Date of filing: 27.10.2022
(51) Int. Cl.: A61B 1/00, A61B 1/12, A61B 1/253

(54) **DENTAL HANDPIECE**

(30) Priority: 27.10.2021 RU 2021131532
(71) Applicant: Medrobotech Ltd, Limassol, 3031 (CY)
(72) Inventor: LIPPGARDT, Ivan Georgievich, Moscow, 115404 (RU); SOLOVYKH, Evgenii Anatol'evich, Moscow, 125362 (RU); ZADUROV, Sergey Igorevich, Moscow, 117534 (RU); BLINKOV, Valentin Alekseevich, Moscow, 111401 (RU); TERKULOVA, Elena Vadimovna, Moscow, 127422 (RU); SOLOVYKH, Anastasiya Evgen'evna, Moscow, 105094 (RU)
(74) Representative: Koudine, Andreï
(86) International application number: PCT/IB2022/060356
(87) International publication number: WO 2023/073616

(57) **Abstract**

The invention relates to a dental handpiece including:
• a handle (6),
• a housing comprising an optical emission source,
• an optical sensor (4) adapted to be connected to visualization devices.

According to the invention, the housing comprises a transparent shielding window (3) adapted to shield the optical sensor (4). The dental handpiece additionally comprises a device for supplying compressed air on the transparent shielding window (3). The housing additionally comprises an aperture for supplying compressed air to the transparent shielding window (3), said aperture being connected to the device for supplying compressed air. A diffuser (16) is located in the aperture, said diffuser being designed so as to generate an air jet forming an air curtain in front of the transparent shielding window (3).

## Description

The present invention relates to dentistry equipment, and more specifically to dental handpieces comprising a handle and a housing with an optical emission source represented by at least one light-emitting diode connected to an electric power supply, said handpiece having an optical sensor adapted to be connected to visualization devices, said handpiece can be used by dentists for dental treatment.

A known example of prior art is a dental handpiece comprising a handle and a housing with an optical emission source represented by at least one light-emitting diode connected to an electric power supply, said handpiece having an optical sensor adapted to be connected to visualization devices, see description of the utility model RU114603 published in 2012.

Said device is the closest art versus the claimed invention and is considered as a prototype. Thus, the claimed invention shall be described in terms of differences from the prototype.

A drawback of said device is that it has a video camera (an optical sensor) which allows to project an image of the mouth cavity to an external screen but cannot ensure that no particles from the mouth cavity get on its surface during dental treatment. Indeed, certain tools, such as drills, can produce solid fragments flying at high speeds that are capable of crossing over an ambient air and getting on the video camera.

The present invention mainly aims to offer a dental handpiece comprising a handle and a housing with an optical emission source represented by at least one light-emitting diode connected to an electric power supply, said dental handpiece having an optical sensor adapted to be connected to visualization devices, said dental handpiece being capable of ensuring reliable protection of the optical sensor by way of an air curtain, which is a stated technical problem.

For this purpose
- the housing has a transparent shielding window of the optical sensor,
- the handpiece has a device for supplying compressed air on the transparent shielding window,
- the housing has an aperture for supplying compressed air to the transparent shielding window connected to the device for supplying compressed air,
- a diffuser is located in the aperture, said diffuser being designed so as to generate an air jet which forms an air curtain in front of the shielding window.

In addition from simply blowing on the transparent shielding window of the optical sensor to protect it from fogging, these useful features make it possible to create a powerful air curtain to protect said shielding window from contamination with solid particles and liquid droplets produced during a preparation of the patient's living tissues, that is, to protect the shielding window from getting any particles on external optical components, in other words, to ensure reliable protection of the optical sensor due to implementation of the air curtain.

There is a possible optional embodiment of the invention in which the diffuser is located from a working end of the housing and is designed so as to direct the air jet away from the housing end (in the direction of the handle).

These useful features make it possible to propose an optional form of the dental handpiece, wherein the optical sensor is located (along the handle) between the drill bit and the handle. Under such conditions, the air jet is directed tangentially to the shielding window away from the working end of the housing, that is, under operational conditions, away from a patient's mouth to help an operator in handling the drill bit and to improve cleaning of the patient's mouth cavity.

Also, there is a possible embodiment of the invention in which the diffuser is located from a working end of the housing and is designed so as to direct the air jet away from the handle. In these conditions, the housing has a protrusion located from the working end, said protrusion having a surface shape which turns the air jet to 120°-140°.

These useful features make it possible to propose an alternative optional form of the dental handpiece, wherein the drill bit is located between the handle and the optical sensor. The air jet is directed tangentially onto the shielding window and away from the handle along the (end of the) housing, then gets rotated by the protrusion, that is, under operational conditions, away from a patient's mouth. This expands the functional capabilities of the dental handpiece and creates an additional convenience for the drill bit operator.

In addition, since the housing has the protrusion located from the working end, said protrusion having a surface shape which turns the air jet to 120°-140°, the formed jet consists of two echelons with differently directed flows. Such complex flow of the high-speed jets makes it possible to increase an efficiency of interception of flying particles by extending their range in velocities and densities. This additionally prevents contamination of the optics.

There is another possible embodiment of the invention in which the diffuser is located from a working end of the housing and is oriented perpendicularly to the handle. Said diffuser is designed so as to direct the air jet sideways from the handle. In these conditions, the housing has a protrusion located from the working end, said protrusion having a surface shape which turns the air jet to 120°-140°.

These useful features make it possible to propose an optional form of the dental handpiece, wherein the optical sensor is located laterally to the drill bit, and the air jet is directed from a central axis of the housing and is rotated similarly to the embodiement described above.

Finally, there is a possible embodiment of the invention in which the diffuser is designed so as to generate the air jet having an outflow speed ranging from 6 m/s to 50 m/s, said outflow speed forming an angle from 0° to 15° with a plane of the transparent shielding window.

These useful features make it possible to propose an alternative optional form of the diffuser with target specifications and ensuring a maximum protection effect for the optical sensor.

Other distinguishing features and advantages of the invention are readily apparent from the description below which includes for illustration but is not limited to the following features, with reference to the figures attached, where:
- figure 1 represents a vertical longitudinal cross-section of a dental handpiece (along a length of a handle, side view) according to a first embodiment of the invention, wherein an optical sensor is located (along the length of the handle) between a drill bit and the handle;
- figure 2 represents a vertical transversal cross-section of the dental handpiece (across its handle, N-N plan view) according to the first embodiment of the invention, as shown in figure 1;
- figure 3 represents a vertical longitudinal cross-section of a dental handpiece (along a length of a handle, side view) according to a second embodiment of the invention, wherein a drill bit is located (along the length of the handle) between an optical sensor and the handle;
- figure 4 represents a vertical transversal cross-section of the dental handpiece (across its handle, B-B plan view) according to the second embodiment of the invention, as shown in figure 3;
- figures 5 and 6 represent vertical transversal (across a handle) cross-sections of the dental handpiece according to a third embodiment of the invention, wherein an optical sensor is oriented perpendicularly to the handle of the housing, and, consequently, a transparent shielding window receives an air flow sideways from a longitudinal axis of the handle;
- figure 7 represents an element A1 of the dental handpiece, said element representing an enlarged view of main assemblies according to the first embodiment of the invention, as shown in figures 1 and 2;
- figure 8 represents an element A2 of the dental handpiece, said element representing an enlarged view of main assemblies according to the second embodiment of the invention, as shown in figures 3 and 4;
- figure 9 represents a vertical longitudinal cross-section view of a working end part of the dental handpiece housing (along the length of its handle, side view) that demonstrates an example with an air jet turning angle of 120 degrees when a protrusion is inclined 80 degrees;
- figure 10 represents a vertical longitudinal cross-section view of a working end part of the dental handpiece housing (along a length of its handle, side view) that demonstrates an example with an air jet turning angle of 140 degrees when a protrusion is inclined 60 degrees;
- figure 11 represents a vertical longitudinal cross-section view of a diffuser part that enables an outflow speed of 3 m/s with an equivalent round section diameter of 2,8 mm;
- figure 12 represents a vertical longitudinal cross-section view of a diffuser part that enables an outflow speed of 50 m/s with an equivalent round section diameter of 0,7 mm;
- figure 13 represents a vertical longitudinal cross-section view of a working end part of the dental handpiece housing (along a length of its handle, side view) that demonstrates an example with an angle 0° between a direction of an air curtain outflow and a transparent shielding window plane 3, when an aperture angle of a diffuser 16 outlet section is 0°;
- figure 14 represents a vertical longitudinal cross-section view of a working end part of the dental handpiece housing (along a length of its handle, side view) that demonstrates an example with an angle 15° between a direction

of an air curtain outflow and a transparent shielding window plane 3, when an aperture angle of a diffuser 16 outlet section is 30.

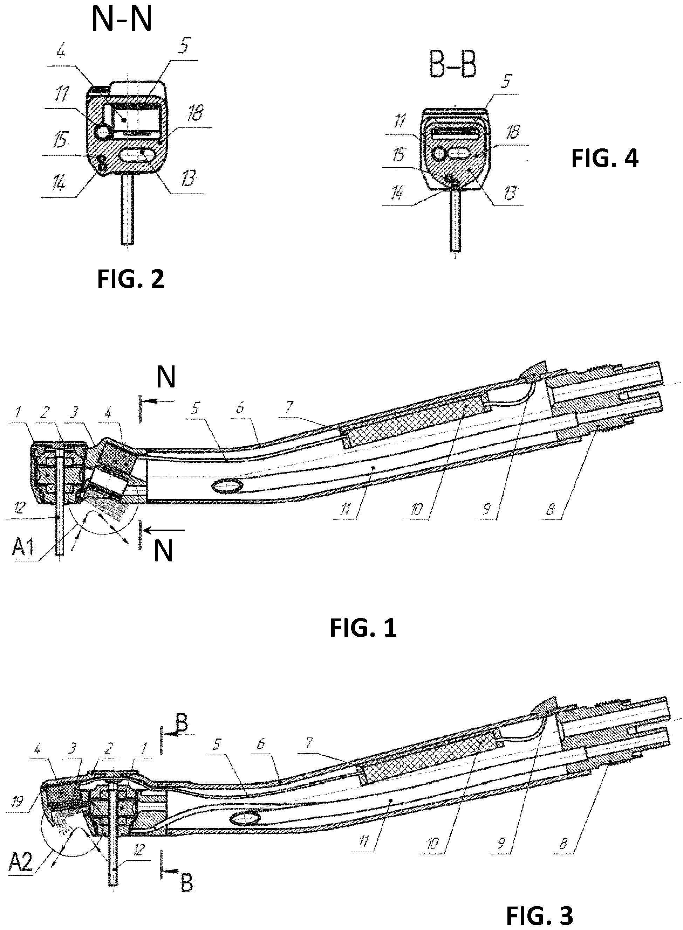

Figures 1 to 14 indicate:
1 - Rotor group.
2 - Collet clamp button.
3 - Transparent shield glass (window).
4 - Optical sensor (camera).
5 - Optical sensor sleeve.
6 - Handpiece handle.
7 - Board latch.
8 - Midwest 4 adapter.
9 - Connector socket.
10 - Camera module board.
11 - Compressed air supplying tube.
12 - Drill bit.
13 - Exhaust air vent.
14 - Water feed to a sprayer.
15 - Air feed to a sprayer.
16 - Diffuser.
17 - Light-emitting diode.
18 - Handpiece housing.
19 - Handpiece cover.

A compressed air source (dental set compressor) is not shown in the figures.

According to figures 1 to 14, a dental handpiece comprises a handle 6 and a housing 18 with an optical emission source represented by at least one light-emitting diode 17 connected to an electric power supply. Said handpiece having an optical sensor 4 adapted to be connected to visualization devices, not shown in the figures.

The housing 18 has a transparent shielding window 3 of the optical sensor. The handpiece has a device for supplying compressed air on the transparent shielding window, also shown as a compressed air supplying tube 11. The housing 18 has an aperture for supplying compressed air to the transparent shielding window connected to the device for supplying compressed air. A diffuser 16 is located in the aperture, said diffuser being designed so as to generate an air jet forming an air curtain before the transparent shielding window 3.

In a first embodiment, the diffuser 16 is located from a working end of the housing 18 and is designed so as to direct the air jet away from the working end of the housing 18 towards the handle (that is, a diffuser outlet is directed to the handle): see figures 1 and 2.

In a second embodiment, the diffuser 16 is located from a working end of the housing 18 and is designed so as to direct the air jet away from the handle 6, that is, an outlet of the diffuser is directed away from the handle (along its length). The housing 18 comprises a protrusion located from the working end, said protrusion having a surface shape which turns the air jet to 120°-140°: see figures 3-4, 9-10.

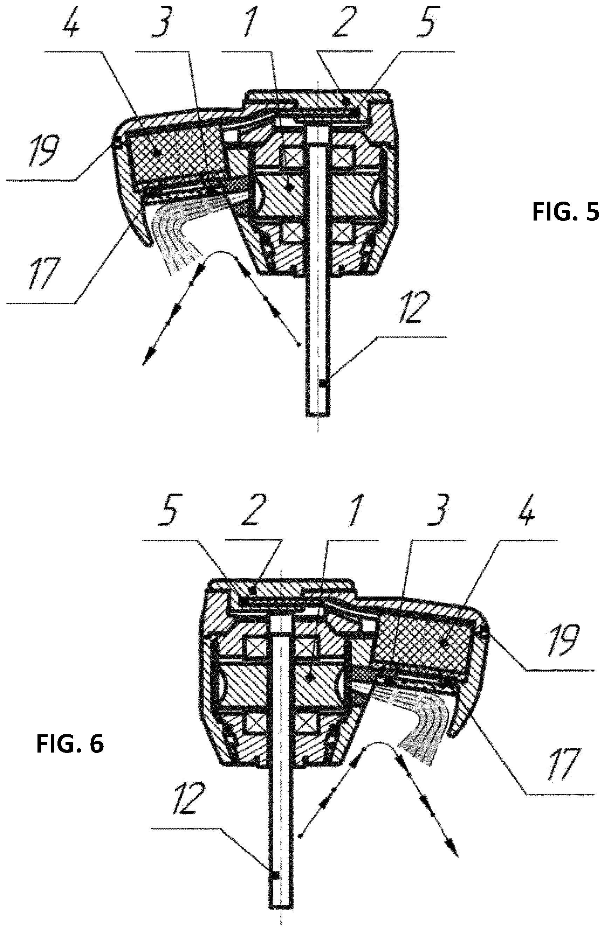

In a third embodiment, the diffuser 16 is located from the transparent shielding window 3 and oriented perpendicularly to the handle 6. The diffuser 16 is designed so as to direct the air jet sideways from the handle 6 of the housing 18, that is, an outlet of the diffuser 16 is directed sideways from the handle 6. The housing 18 comprises a protrusion located from the working end, said protrusion having a surface shape which turns the air jet to 120°-140°: see figures 5-6, 9-10.

The diffuser 16 is also preferably designed so as to generate the air jet with the outflow speed ranging from 6 m/s to 50 m/s and forming an angle from 0° to 15° with a plane of the transparent shielding window 3.

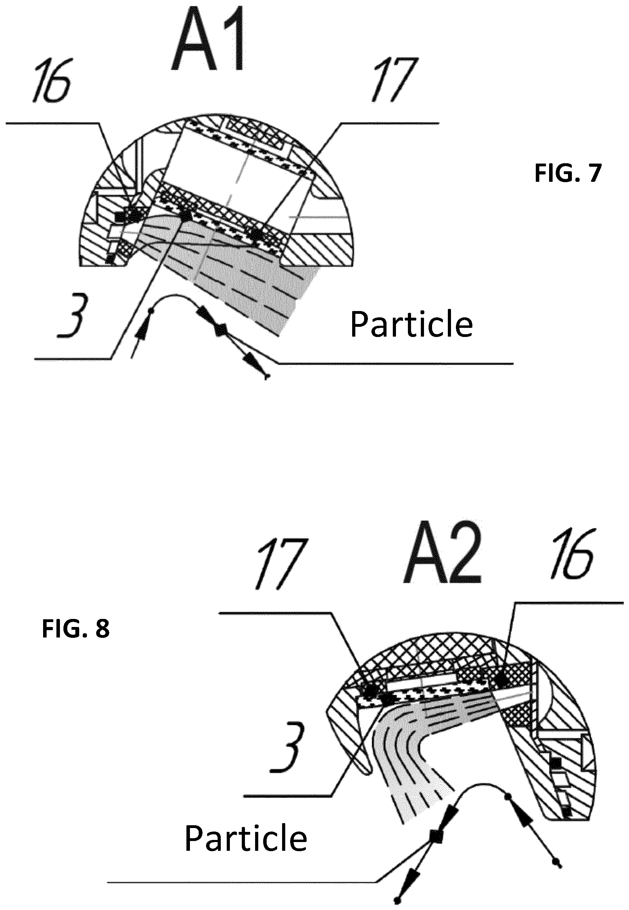

Said dental handpiece is also designed with several air supply options: direct (without turning the air curtain flow as shown in the figures 1, 7) and reverse (with turning of the air curtain, as shown in the figures 3, 5-6, 8-10), and in different locations of the optical sensor 4 relative to a drill bit 12 (in front of, behind, to the right, to the left, relative to a longitudinal axis of the drill bit 12), as is evident from figures 1-6.

The dental handpiece operates as follows. Below is the most exhaustive example of the embodiment of the invention, however, said example does not limit other applications of the invention.

**Example.** A compressed air is supplied from a dental unit compressor through a compressed air supplying tube 11 as a channeled air jet to a housing 18. When the air passes through the housing 18, it goes to a directional diffuser 16 that forms a flat jet directed at an angle from 1 to 15 degrees to the housing 18 surface with an outflow speed ranging from 6 m/s to 50 m/s. The high-speed jet does not allow foreign particles having densities and speeds typical for dental interventions pass through its core and moves them away from the transparent shielding window 3 of the optical sensor (camera) 4.

Quantitative attributes of the technical parameters such as an the air jet turning angle, the air jet outflow speed and the angle between the air jet outflow and the transparent shielding window 3 plane in the range of continuously changing variables can be adjusted by varying a protrusion and the diffuser 16 geometry.

The selection of a specific range of technical parameter values is made experimentally and depends on the efficiency of optical sensor (camera) 4 shielding window blowing. Blowing efficiency control method shall be a visual control of readability of the image transferred from the optical sensor 4 and contaminated with artifacts due to foreign particles and liquid droplets getting onto the transparent shielding window 3. The number of image artifacts shall be assumed as a criterion of blowing efficiency in four tiers:
- "none" - no visible artifacts from foreign particles and liquid droplets;
- "moderate" - foreign particles and liquid droplets are not retained on the surface of the transparent shielding window 3 and do not affect the readability of the transferred image;
- "medium" - foreign particles and liquid droplets remain on the surface of the transparent shielding window 3 and affect the image transferred from the optical sensor 4. However, readability remains sufficient to direct the dental handpiece in the operating environment;
- "severe" - foreign particles and liquid droplets remain on the surface of the transparent shielding window 3, making the image from the optical sensor 4 unreadable and not suitable for correct orientation of the dental handpiece in the operating environment.

Air jet rotation angle is adjusted in the range from 120° to 140° by adjusting the shape of the protrusion surface directed at the diffuser 16. A protrusion incline angle shall be the geometrical parameter for an airjet turning angle. The protrusion incline angle is found between the tangential line to the margin of the concave surface and the plane of the transparent shielding window 3.

See Table 1 for the relationship between the air jet turning angles, image artifact grade, and the above geometrical parameter (i.e. protrusion incline angle).

**Table 1. Dependence of air jet rotation angles and image artifact grade from protrusion incline angle.**

| **No** | **Air jet turning angle, degree** | **Protrusion incline angle, degree** | **Image artifact grade** |
|---|---|---|---|
| 1 | 115 | 85 | Medium |
| 2 | 120 | 80 | Moderate |
| 3 | 125 | 75 | Moderate |
| 4 | 130 | 70 | None |
| 5 | 135 | 65 | None |
| 6 | 140 | 60 | None |

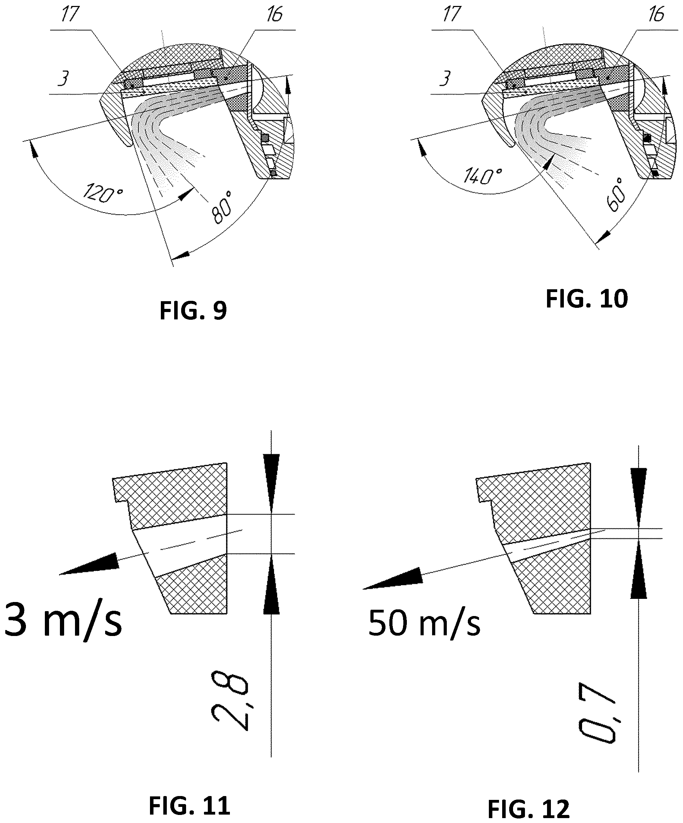

See figures 9 and 10 for the examples of the embodiments of the invention with border values in the specified range, namely:
- figure 9 shows the example with the air jet rotation angle of 120 degrees which results in moderate image artifact grade, with the protrusion incline angle of 80 degrees;
- figure 10 shows the example with the air jet rotation angle of 140 degrees which results in no image artifacts (grade "none"), with the protrusion incline angle of 60 degrees.

Air jet outflow was adjusted in the range from 6 m/s to 50 m/s by adjusting the diffuser 16 outlet cross-section area. The geometrical parameter characterizing air jet outflow speed was round section diameter with the same area as the diffuser 16 outlet cross-section. See Table 2 for the relationship between air jet outflow, image artifact grade, and the above geometrical parameter.

**Table 2. Dependence of air jet outflow speed and image artifact grade from equivalent round section diameter.**

| **No** | **Air jet outflow speed, m/s** | **Equivalent round section diameter, mm** | **Image artifact grade** |
|---|---|---|---|
| 1 | 3 | 2,8 | Severe |
| 2 | 6 | 2,0 | Medium |
| 3 | 10 | 1,6 | Moderate |
| 4 | 20 | 1,1 | Moderate |
| 5 | 30 | 0,9 | Moderate |
| 6 | 40 | 0,8 | None |
| 7 | 50 | 0,7 | None |

See Figures 11 and 12 for the examples of the embodiments of the invention with border values in the specified range, namely:
- figure 11 shows an example with an air jet outflow speed of 3 m/s and, respectively, severe image artifact grade, with an equivalent round section diameter of 2,8 mm;
- figure 12 shows an example with an air jet outflow speed of 50 m/s and, respectively, no image artifacts, with an equivalent round section diameter of 0,7 mm.

The angle between the air curtain outflow direction and shielding window plane was adjusted in the range from 0 to 15 degrees by adjusting the diffuser 16 outlet cross-section aperture angle. The diffuser 16 outlet cross-section aperture angle was assumed as the characteristic geometrical parameter. See Table 3 for the dependence of the angle between the direction of the air curtain outflow direction and shielding window plane and image artifact grade on the above geometrical parameter.

**Table 3. Dependence of the angle between the direction of the air curtain outflow direction and shielding window 3 plane and image artifact grade on the diffuser 16 outlet cross-section aperture angle.**

| **No** | **Angle between the direction of the air curtain outflow direction and shielding window 3 plane, degrees** | **Diffuser 16 outlet cross-section aperture angle, degrees** | **Image artifact grade** |
|---|---|---|---|
| 1 | 0 | 0 | None |
| 2 | 2,5 | 5 | None |
| 3 | 5 | 10 | Moderate |
| 4 | 7,5 | 15 | Moderate |
| 5 | 10 | 20 | Medium |
| 6 | 12,5 | 25 | Medium |
| 7 | 15 | 30 | Medium |
| 8 | 17,5 | 35 | Severe |

See Figure 3 for the examples of the embodiments of the invention with border values in the specified range, namely:

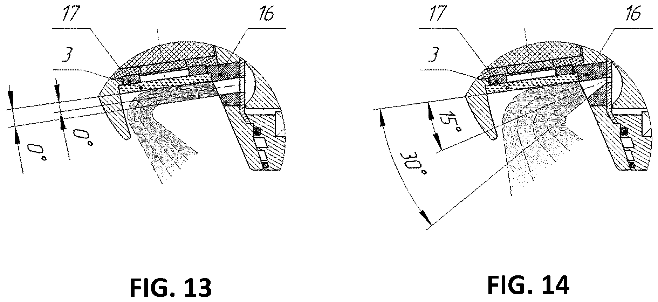

- figure 13 shows an example with the angle between the direction of the air curtain outflow direction and shielding window 3 plane equaling 0° and no image artifacts, respectively, at the diffuser 16 outlet cross-section aperture angle of 0°;
- figure 14 shows an example with the angle between the direction of the air curtain outflow direction and shielding window 3 plane equaling 15° and medium image artifact grade, respectively, at the diffuser 16 outlet cross-section aperture angle of 30°.

Therefore, (see figures 1-14), the invention relates to a dental handpiece comprising:
- a handle 6,
- a housing 18 comprising an optical emission source represented by at least one light-emitting diode 17 connected to an electric power supply,
- an optical sensor 4 adapted to be connected to visualization devices.
According to the invention, the housing 18 comprises a transparent shielding window 3, adapted to shield the optical sensor 4. The dental handpiece additionally comprises a device for supplying compressed air to the transparent shielding window 3. The housing 18 additionally comprises an aperture for supplying compressed air to the transparent shielding window 3, said aperture being connected to the device for supplying compressed air. A diffuser 16 is located in the aperture, said diffuser being designed so as to generate an air jet forming an air curtain before the transparent shielding window 3.

In a first embodiment of the invention (Figures 1-2, 7), the diffuser 16 is located from a working end of the housing 18. The diffuser 16 is designed so as to direct the air jet away from the working end of the housing 18 longitudinally towards the handle 6.

In an alternative second embodiment of the invention (Figures 3-4, 8), the diffuser 16 is located from the working end of the housing 18. Under these conditions, the diffuser 16 is designed so as to direct the air jet away from the handle 6 (that is, along the length of the handle 6). The housing 18 has a protrusion located from the working end. The protrusion has a surface shape which turns the air jet outgoing from the diffuser 16 in a first range from 120° to 140° (Figures 9 to 10).

In an alternative second embodiment of the invention (Figures 5-6), the diffuser 16 is located from the working end of the housing 18 and oriented perpendicularly to the handle 6. The diffuser 16 is designed so as to direct the air jet sideways from the housing 18 handle 6. The housing 18 comprises a protrusion located from the working end. The protrusion has a surface shape which turns the air jet outgoing from the diffuser 16 by the first angle in the first range from 120° to 140° (Figures 9-10).

Preferably, the diffuser 16 is designed so as to generate the air jet with an outflow speed ranging from 6 m/s to 50 m/s (Figures 11-12). This air jet outflow forms a second angle with a plane of the transparent shielding window 3, said second angle being comprised in a second range from 0° to 15° (Figures 13 to 14).

The claimed dental handpiece may be implemented by a person skilled in the art in practice and ensures that the claimed objectives are met after implementation, which leads to the conclusion that the invention meets the requirement of "industrial applicability".

A series of real-life tests was performed to confirm the operability of the proposed dental handpiece. As an example, the preparation of the upper 8^{th} tooth with a compound cavity was recorded on the video. The preparation was made using a standard air turbine handpiece with standard water spraying of the intervention area. The sensor shielding window was located in the range from 25 mm to 28 mm from the preparation area at the angle of 60 to 70 degrees. No additional optics protection measures were taken, only standard-grade dental equipment and tools specific for such interventions were used. The video shows that, under the conditions of active water supplying and high-intensity spraying, the transparent shielding window 3 is not polluted by solid enamel and dentine particles cut by rotating tools, and that the camera 4 continues operation throughout the intervention.

Therefore, these novel features make it possible to achieve the claimed technical results, namely to ensure reliable protection of the optical sensor by implementing an air curtain. Said dental handpiece is also designed with several air feed options: direct and reverse (with turning of the air curtain flow) and in different locations of the optical sensor 4 relative to the drill bit 12 (in front of, behind, to the right, to the left, relative to the drill bit 12 longitudinal axis).

## Claims

1. A dental handpiece including:
• a handle (6),
• a housing (18) comprising an optical emission source represented by at least one light-emitting diode (17) connected to an electric power supply,
• an optical sensor (4) adapted to be connected to visualization devices,
**characterized in that** the housing (18) comprises a transparent shielding window (3) adapted to shield the optical sensor (4), **in that** the dental handpiece additionally comprises a device for supplying compressed air on the transparent shielding window (3), **in that** the housing (18) additionally comprises an aperture for supplying compressed air to the transparent shielding window (3), said aperture being connected to the device for supplying compressed air, and **in that** a diffuser (16) is located in the aperture, said diffuser being designed so as to generate an air jet which forms an air curtain in front of the transparent shielding window (3).

2. The dental handpiece according to claim 1, **characterized in that** the diffuser (16) is located from a working end of the housing (18), and **in that** the diffuser (16) is designed so as to direct the air jet away from the working end of the housing (18) longitudinally towards the handle (6).

3. The dental handpiece according to claim 1, **characterized in that** the diffuser (16) is located from a working end of the housing (18), **in that** said diffuser (16) is designed so as to direct the air jet longitudinally away from the handle (6), **in that** the housing (18) comprises a protrusion located from the working end, and **in that** the protrusion has a surface shape which turns the air jet outgoing from the diffuser (16) to a first angle comprising in a first range from 120° to 140°.

4. The dental handpiece according to claim 1, **characterized in that** the diffuser (16) is oriented perpendicularly to the handle (6), **in that** the diffuser (16) is designed so as to direct the air jet sideways from the handle (6), **in that** the housing (18) comprises a protrusion located from the working end, and **in that** said protrusion has a surface shape which turns the air jet outgoing from the diffuser (16) to a the first angle comprising in a first range from 120° to 140°.

5. The dental handpiece according to claim 1, **characterized in that** the diffuser (16) is designed so as to generate the air jet with an outflow speed ranging from 6 m/s to 50 m/s, and **in that** the outflow speed forms a second angle with a plane of the transparent shielding window (3), said second angle being comprised in a second range from 0° to 15°.
